(19) Europäisches Patentamt / European Patent Office / Office européen des brevets

(11) **EP 4 751 705 A1**

(12) **EUROPEAN PATENT APPLICATION**

(43) Date of publication:
03.06.2026 Bulletin 2026/23

(21) Application number: 24216436.6

(22) Date of filing: **29.11.2024**

(51) International Patent Classification (IPC):
$A61K\ 8/19^{(2006.01)}$  $A61K\ 8/49^{(2006.01)}$
$A61K\ 8/64^{(2006.01)}$  $A61Q\ 5/00^{(2006.01)}$
$A61K\ 8/60^{(2006.01)}$  $A61K\ 31/7034^{(2006.01)}$
$A61K\ 33/34^{(2006.01)}$  $A61P\ 17/00^{(2006.01)}$

(52) Cooperative Patent Classification (CPC):
(C-Sets available)
**A61K 8/602; A61K 8/19; A61K 8/49; A61K 8/64;
A61K 31/7034; A61K 33/34; A61P 17/00;
A61Q 5/00; A61Q 5/006;** A61K 2800/58     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **SkyLab AG**
**1066 Epalinges (CH)**

(72) Inventors:
• **Belous, Elena**
**121309 MOSCOW (RU)**
• **Ivanova, Angelina**
**LONDON, NW61NE (GB)**

(74) Representative: **Glawe, Delfs, Moll**
**Partnerschaft mbB**
**Hopfenmarkt 33**
**20457 Hamburg (DE)**

(54) **N2,3,5,4'- TETRAHYDROXYSTILBENE 2-O-B -D-GLUCOSIDE AND COPPER COMPOUNDS FOR MELANIN PROLIFERATION, GREY HAIR PIGMENTATION STIMULATION AND ANTI-DANDRUFF THERAPY**

(57) The invention relates to a composition comprising 2,3,5,4'-Tetrahydroxystilbene 2-O-β-D-glucoside and at least one compound comprising copper. Said composition id for medical and non-medical use, including for preventing and/or reversing hair greying and/or dandruff in a subject.

Molecular Modeling of THSG-MC1R Affinity

Fig. 1

**(Cont. next page)**

EP 4 751 705 A1

(52) Cooperative Patent Classification (CPC): (Cont.)

C-Sets
**A61K 31/7034, A61K 2300/00;**
**A61K 33/34, A61K 2300/00**

**Description**

FIELD OF THE INVENTION

The present invention pertains to the application of 2,3,5,4'-Tetrahydroxystilbene 2-O-β-D-Glucoside and its combination with copper compounds to induce melanin synthesis in humans. The invention includes the use of these agents to prevent or reverse hair graying and as an anti-dandruff treatment. This invention falls within the fields of cosmetic and dermatological therapies and is intended for application in hair and scalp care products. Both 2,3,5,4'-Tetrahydroxystilbene 2-O-β-D-Glucoside and its combinations with copper compounds demonstrate stability and are fully compatible for incorporation into cosmetic and pharmaceutical formulations.

BACKGROUND OF THE INVENTION

The process of graying, or achromotrichia, is a natural aspect of the body's aging mechanism. Globally, 6-23% of individuals exhibit up to 50% gray hair by the age of 50 [1]. The depigmentation of hair typically initiates between the ages of 30 and 40 in individuals of European descent, around 40 in Asians, and between 40 and 50 in Africans [2-4]. Early-onset graying is defined as onset before 20 years in Caucasians, before 25 in Asians, and before 30 in Africans [4,5].

In men, gray hair commonly begins at the temples and side-burns, later appearing on the crown and back of the head. In women, graying generally starts at the hairline, progressively extending towards the crown. While the onset age of gray hair is genetically influenced, early graying does not necessarily correlate with accelerated progression [6].

Certain reversible factors contribute to premature graying, such as nutrient deficiencies. Deficiencies in vitamin B12, iron, and copper, along with severe protein insufficiency, are linked to hypopigmentation of hair [3,7,8]. Risk factors include vegetarian diets and atopic conditions [9].

The human hair shaft comprises two main concentric regions: the inner cortex and the surrounding outer cuticle. Some hair shafts also contain a central medulla [2]. Histologically, five to six distinct subpopulations of melanocytes have been identified. Melanogenically active melanocytes are located at the hair exit point on the scalp, within the sebaceous gland, and around the dermal papilla within the hair bulb. Additionally, undifferentiated, inactive melanocytes are found in the upper hair follicle near the arrector pili muscle attachment point, along the outer root sheath of the follicle, and within the hair bulb matrix [10]. Active melanocytes synthesize and transfer melanin to cortical keratinocytes in the hair shaft; a minor portion of melanin is transported to the medulla, with even less reaching the cuticle [4]. The role of inactive melanocytes remains poorly understood, although they are believed to serve as a stem cell reservoir, capable of differentiating into melanin-producing cells in response to skin injury [2,4].

Hair melanogenesis is closely linked to the stages of the hair growth cycle. Hair is actively pigmented during the anagen phase (growth phase) but not in the catagen (resting phase) or telogen (shedding phase) stages [3, 4]. In humans, anagen generally lasts around 3.5 years, requiring a sustained population of follicular melanocytes capable of producing significant amounts of melanin [11]. Follicular melanocytes, characterized by a larger Golgi apparatus and rough endoplasmic reticulum, are larger than epidermal melanocytes, facilitating the formation of larger melanosomes [12]. Follicular melanin also degrades more slowly than epidermal melanin, resulting in consistent pigmentation along the distal and proximal ends of the hair shaft [10]. The specific hair color is determined by the type of melanin pigment produced by follicular melanocytes, such as the black-brown eumelanin or the red-yellow pheomelanin [3].

A variety of factors stimulate melanogenesis at the follicular level, including melanocyte-stimulating hormone (MSH), adrenocorticotropic hormone (ACTH), endothelin-1, prostaglandins, leukotrienes, neutrophils, fibroblast growth factor, nitric oxide, and catecholamines [5]. Inhibitors of melanogenesis include sphingolipids, bone morphogenetic protein 4, and autoimmune processes, such as those observed in vitiligo and alopecia areata [2,3,10,13]. Certain compounds or medical conditions can influence the production of melanogenic factors, thereby altering hair pigmentation. Conditions sometimes associated with hair darkening include Addison's disease, neurodermatitis, late-onset porphyria cutanea, and inflammatory scalp diseases [2,14-16]. Disorders linked to hair lightening or graying include cystic fibrosis, celiac disease, hyperthyroidism/hypothyroidism, vitiligo, alopecia areata, and genetic syndromes such as Werner syndrome, Louis-Bar syndrome, Waardenburg syndrome, and Griscelli syndrome [2,13,17,18].

The amino acid tyrosine, essential for catecholamine synthesis, and its hydroxylated product, DOPA (L-Dihydroxyphenylalanine), serve as the starting points in the biosynthesis of melanin pigments. Melanin is synthesized through a series of enzyme-catalyzed reactions. The red-yellow melanin pigment, pheomelanin - a polymer soluble in alkaline solutions - is formed by the oxidative polymerization of cysteinyldopa, resulting from the condensation of the amino acid cysteine with dopaquinone. Eumelanin, an insoluble polymer that ranges in color from brown to black, is produced through the oxidative polymerization of 5,6-dihydroxyindole. The hydroxylation of L-tyrosine to L-DOPA is catalyzed by tyrosinase (TYR) and a series of tyrosinase-related proteins [23].

Tyrosinase, a type 3 membrane-bound protein [24], is an essential enzyme in melanogenesis. It is localized within the membrane of melanosomes, the organelles specific to melanocytes. This enzyme is synthesized within the endoplasmic reticulum and then packaged in the Golgi apparatus before being transported to melanosomes - the site of melanin synthesis. Tyrosinase activity correlates directly with melanin production levels, and both melanosome formation and tyrosinase activity are stimulated by L-tyrosine [25]. The concentrations of L-tyrosine and L-DOPA play a critical role in maintaining the homeostasis of the melanogenic system, with melanocytes orchestrating this regulatory balance [26].

Azelaic acid is known to inhibit DNA synthesis and mitochondrial enzyme activity, impacting tyrosinase and reducing production of free radicals that contribute to skin hyperpigmentation [27]. Due to its direct cytotoxic effects on abnormal melanocytes, azelaic acid is beneficial in treating hyperpigmentation without affecting normal skin pigmentation or causing depigmentation.

In the human brain, neuromelanin is formed through the oxidation of dopamine to dopaquinone, a reaction catalyzed by tyrosinase. Excessive dopaquinone formation is associated with neural disorders and cell death [28]. Thus, tyrosinase is implicated in neurodegeneration related to Parkinson's disease [29] and Huntington's chorea [30, 31]. In addition to tyrosinase, two other proteins - dopachrome tautomerases - are involved in the conversion of tyrosine into melanin pigments. These proteins, designated as TRP-1 and TRP-2, are likewise located in melanosomes and are crucial for catalyzing the reactions that produce eumelanin. TRP-1 functions as a peroxidase and provides protection against oxidative stress [32, 33] .

The onset of gray hair is associated with a reduction in melanocyte numbers, resulting from either defects in melanocyte stem cells or depletion of the follicular stem cell population [2-4,19]. A common cause of follicular melanocyte death is oxidative stress, triggered by reactive oxygen species (ROS) production from hydrogen peroxide accumulation, a byproduct of the hair growth cycle, or ultraviolet (UV) radiation [2,3,19,20]. Melanocytes naturally produce antioxidants - such as the apoptosis regulator BCL-2, catalase, and methionine sulfoxide reductase - to defend against ROS damage. However, these protective molecules are deficient in graying hair follicles [19,20]. For instance, studies on BCL-2-deficient mice demonstrated graying by the second hair growth cycle [19, 20]. Interestingly, melanocytes in the outer root sheath are less susceptible to ROS damage and may serve as a pigment source for hair color restoration [19]. Environmental pollution, emotional stress, alcohol consumption, and smoking - factors that exacerbate oxidative stress - are also associated with premature graying [21, 22].

Anti-inflammatory agents are sometimes employed to counteract hair graying. While most cases reported are based on individual observations, the literature includes one prospective cohort study and a retrospective study involving 39 patients.

Pavithran [34] documented that psoralen combined with UV light (PUVA therapy) induced repigmentation of gray hair, particularly in patients with premature graying. This concept originated from clinical experience with psoriasis treatment. Consequently, a prospective study was conducted on healthy patients aged 10-20 years with premature graying (n = 37). After 13 months of treatment, 46% of these patients exhibited complete scalp hair repigmentation with no recurrence over an 8-month follow-up period [35]. Seven additional patients showed partial repigmentation, either with pigment appearing at the proximal ends of gray hair shafts or with diffuse or patchy light-brown pigmentation [36].

In a retrospective study involving men undergoing cisplatin-based chemotherapy for germ cell neoplasms, patients aged 15-54 years exhibited color changes in their regrown hair. Of the 69 patients, 16% reported darkening of hair color [37]. Two patients experienced sustained hair color restoration within 2 years post-chemotherapy.

Other isolated instances of anti-inflammatory agents inducing gray hair repigmentation have also been observed. Retinoic acid receptor activators, such as acitretin and etretinate, were linked to gray hair repigmentation in two patients with pityriasis rubra pilaris and one patient with psoriasis after 6-12 months of treatment [38-40]. In one case, a patient receiving interferon-alpha for chronic hepatitis C reported scalp hair repigmentation, which began two months after treatment initiation and stabilized upon completion of therapy [41]. Case reports on other anti-inflammatory agents known to inhibit pro-inflammatory cytokines, including thalidomide, lenalidomide, adalimumab, cyclosporine, and prednisolone, also linked these drugs to hair repigmentation following 2-24 months of treatment [42-48]. Inflammatory processes within hair follicles can adversely affect hair pigmentation. However, the use of anti-inflammatory agents has not consistently led to hair repigmentation across all patients, indicating that multiple factors influence the graying process.

Stimulating melanogenesis presents an alternative approach for addressing gray hair. Five pharmacological agents believed to stimulate melanogenesis have been documented in one retrospective study and six case reports involving hair repigmentation. In a retrospective study, nine out of 133 patients receiving imatinib for chronic myelogenous leukemia exhibited repigmentation of gray hair within 2-14 months of treatment initiation [49]. Additionally, another tyrosine kinase inhibitor, erlotinib, was reported to induce progressive hair repigmentation in two separate cases of patients with metastatic lung adenocarcinoma within 3 months and 2 years, respectively, following treatment initiation [50-51]. In one case, hair repigmentation associated with erlotinib was observed after the resolution of scalp folliculitis [52]. However, erlotinib has also been associated with androgenic alopecia in certain cases, emerging months after treatment commencement [53].

Color restoration was observed three years after initiating latanoprost eye drops for open-angle glaucoma [54]. Another patient reported hair repigmentation on the scalp 2.5 years after beginning tamoxifen therapy for breast cancer [55]. Lastly, two patients taking levodopa for Parkinson's disease noted diffuse hair repigmentation within 8-9 months of treatment initiation [56-57].

Vitamin and mineral deficiencies are considered among the causes of premature gray hair, thus replenishing such deficiencies may restore hair color. Research of B vitamins supplemented with calcium pantothenate or potassium para-aminobenzoic acid (PABA) represents some of the earliest targeted attempts at gray hair repigmentation. In two healthy patients, successful repigmentation of premature gray hair occurred with high-dose calcium pantothenate (200 mg per day), with initial repigmentation observed within one month of treatment [58]. A subsequent three-year prospective cohort study involving seven women aged 12-31 with premature gray hair reported repigmentation in two patients taking 200 mg per day for three months and in another two patients at a dose of 100 mg [59]. One prospective cohort study and one retrospective study investigated PABA's effectiveness in treating gray hair [60,61]. In 1941, Siev [62] conducted the first documented study of gray hair repigmentation using PABA at a dosage of 200 mg per day in 50 patients with premature or age-related graying. Subjectively noted darkening of hair was observed in all patients after two months of treatment. Another study examined the effects of high-dose PABA (12-24 g/day) on age-related gray hair as part of systemic disease treatment for conditions such as cutaneous lymphoblastoma, dermatomyositis, herpetiform dermatitis, and scleroderma (n = 20) [63]. Seven patients reported hair darkening within 2-10 months of treatment. A prospective study assessing the combined administration of 100 mg calcium pantothenate and 200 mg PABA daily for gray hair treatment (involving 27 patients with age-related gray hair and 6 with premature gray hair) reported visually noticeable hair color change in two patients (both with age-related graying) and minor color change in seven after eight months of supplementation [64]. This study also noted that repigmented hair reverted to gray following supplementation cessation.

The market offers a range of active ingredients aimed at combating gray hair, with Greyverse being a notable example. Greyverse is a water-glycerin solution containing synthetic palmitoyl tetrapeptide-20 amide, derived from a reaction between palmitic acid and synthetic tetrapeptide-20, which is composed of amino acid residues including arginine, histidine, phenylalanine, and tryptophan. Its efficacy is supported by extensive research, demonstrating a 66% increase in melanogenesis, a 30% reduction in hydrogen peroxide levels, a 5.3% decrease in the overall number of gray hairs, and a 30% reduction in their density after three months of application [65]. However, its use is limited by a high cost - $2000 per kilogram - and stability issues within formulations under varying pH levels and storage temperatures. In a 100 ml product formulation containing 2% Greyverse, the contribution to production cost is approximately $5. Research findings indicate no effect on hair growth stimulation, focusing solely on repigmentation.

Another product available on the market is Darkenyl. The active composition of Darkenyl includes water, glycerin, acetyl tyrosine, sodium meta-bisulfite, glycine, larch wood extract (Larix Europaea Wood Extract), zinc chloride, and camellia leaf extract (Camellia Sinensis Leaf Extract). The key active ingredients are synthetic acetyl tyrosine and natural larch extract (Larix Europaea Wood Extract). Darkenyl promotes the expression of genes associated with stem cell proliferation, hair follicle morphogenesis, and maintenance of the follicular stem cell phenotype. It increases cell proliferation by 30% compared to the positive control. Under oxidative stress conditions, the components significantly boost the expression of genes involved in antioxidant defense, hair renewal, and follicular pigmentation. Reactive oxygen species (ROS) accumulation decreases by 53%, and following oxidative stress exposure, Darkenyl restores the quantity of melanocytes and melanoblasts in the hair follicle by 189%. Melanin production increases by 15% within three days of use. Clinical trials showed that after four months of treatment, Darkenyl reduced the amount of gray hair by an average of 17% compared to the placebo group [66]. Despite its promising results, Darkenyl has limitations, including a high cost - ranging from $50 (serum) to $150 (shampoo) - and the presence of synthetic ingredients, which may deter consumers who prefer natural components. While Darkenyl is free from side effects and drug interactions, its tyrosine content may interact with medications for Parkinson's disease, hypertension, or depression. These conditions are often prevalent in the age group where graying may start, thereby restricting Darkenyl's use for certain consumers who would otherwise be within its target demographic.

2,3,5,4'-Tetrahydroxystilbene 2-O-β-D-Glucoside (THSG) is an active compound derived from the traditional Chinese medicinal plant Polygonum multiflorum Thunb. Pharmacological studies have demonstrated THSG's extensive biological functions in treating conditions such as atherosclerosis, lipid metabolism disorders, vascular and cardiac remodeling, vascular fibrosis, cerebrovascular ischemia, learning and memory impairment, neuroinflammation, Alzheimer's and Parkinson's diseases, diabetic complications, hair growth issues, among various other conditions. Research [67] indicates that THSG induces activation of MITF (Melanocyte Inducing Transcription Factor) and also promotes phosphorylation of CREB (Cyclic AMP Response Element Binding Protein), which binds and activates MITF. Additionally, the activation of p38 MAPK (Mitogen-Activated Protein Kinase) has been shown to play a crucial role in THSG-induced melanogenesis in B16F1 cells by upregulating MITF and tyrosinase expression. Consequently, the pigmentation-enhancing properties of THSG may serve as an adjunct therapy in addressing conditions associated with graying hair. Gray hair management presents a multifaceted challenge, necessitating a comprehensive approach targeting the stimulation of melanogenesis, mitigation of oxidative stress effects, and potentially supplying essential melanin synthesis components. THSG distinguishes itself from other gray hair treatment actives on the market by combining both antioxidant and melanogenesis-stimulating properties along with a natural origin. In contrast, most commercially available actives are synthetic and offer a narrow range of effects, which often increases the number of active ingredients required in formulations. However, a key limitation of THSG is its stability. Data [67] indicates that its half-life at 25°C is 47.57 days at pH 1.5 and only 0.11 days at pH 9.9, presenting formulation challenges.

THSG is also occasionally found within Polygonum multiflorum root extract, which is included in dietary supplements and hair care products. This root has been traditionally used in Chinese medicine for treating and preventing various health conditions. However, its use is limited by potential hepatotoxicity, as various studies have documented its adverse effects on liver health. Cases of abdominal pain, diarrhea, nausea, and vomiting have been reported, linked to anthraquinones like emodin present in the extract [68]. Emodin inhibits cell proliferation and is cytotoxic to human reproductive tissues [69]. Utilizing a single purified component of the extract allows targeted action on the desired effect with increased efficacy, as the substance is isolated from other compounds, thereby eliminating unwanted side effects.

Copper deficiency in the body is associated with hypopigmentation of hair. Copper is included in popular dietary supplements aimed at preventing gray hair and restoring hair color, such as Not today, Grey and Gray Escape™ Advanced Anti-Gray Hair Growth Supplement. An ex vivo study [70] confirmed that a copper tripeptide, composed of a positively charged copper ion and the amino acid residues L-alanyl-L-histidyl-L-lysine Cu, promotes the growth of human hair follicles. This growth-promoting effect is hypothesized to arise from the stimulation of cell proliferation and prevention of apoptosis in dermal papilla cells. Oxidative stress can adversely affect hair, including contributing to the onset of gray hair. Researchers [71] confirmed, using one method, that copper ions adsorbed onto hair from tap water increase hair damage induced by sunlight exposure, particularly through the increased loss of protein during shampooing. However, another method did not show similar effects. Due to the lack of conclusive evidence on the influence of copper compounds on oxidative stress, it is not possible to definitively assert a positive impact of this active ingredient in combating gray hair. The use of copper peptides as an anti-gray hair agent is also described in patent KR20120048830A [72]. However, there is limited research on the efficacy of copper peptides when applied topically to the hair and scalp, and no experimental evidence to confirm their role in melanogenesis stimulation.

The inventors hypothesized that the combined application of THSG and Copper Compounds may support the induction of melanogenesis, provide antioxidant protection, and reduce dandruff. However, this hypothesis is limited by the instability of THSG at varying temperatures and pH levels, as previously noted, the potential oxidative activity of copper ions, and the lack of evidence supporting the melanogenesis-stimulating properties of Copper Compounds when applied topically to the hair and scalp.

INVESTIGATIONS

**Example 1.** In-silico investigation THSG affinity to MC1R protein

1.1 Materials and methods

1.1.1 Investigated ingredients

Table 1. Investigated ingredients in-silico study

| No. | Ingredient |
|---|---|
| 1 | 2,3,5,4' - Tetrahydroxystilbene 2-O-B-D-Glucoside |
| 2 | Copper Tripeptide |
| 3 | Copper Bisglycinate |

1.2 Investigation

The MC1R gene and its encoded receptor protein for melanocyte-stimulating hormone (MSH) are primarily associated with the activation of eumelanin pigment biosynthesis in response to UV radiation. In addition, the MC1R gene is able to regulate the work of other genes associated with melanogenesis. MC1R protein structure was sourced in .cif format from the Cambridge Crystallographic Data Centre. For further computational analysis, the MC1R structure was converted and saved in .xyz format. The ligand structures were obtained from PubChem [73].

1.2.1 Molecular Modeling and Affinity Analysis for Ligand-MC1R Interaction

For the computational studies, a personal computer equipped with an Intel Core i9-13900K CPU operating at 2.3 GHz and 64 GB of RAM was employed. The operating system used was Windows 11, 64-bit. The structural optimization of both MC1R and the ligands was performed using ORCA 5.0.3 software [74]. Subsequently, the ligand-MC1R complexes were manually assembled, and the resulting models were stored in .xyz format for further analysis using ORCA 5.0.3 software [74].

The following quantum chemical approximations were applied in the calculations: the density functional theory (DFT) functional used was PBE0 [75], with dispersion corrections applied via D3(BJ) [76]. Solvation effects were considered using the conductor-like polarizable continuum model (CPCM), with water as the solvent. The basis set utilized was def2-TZVPP [77]. Geometry optimizations were conducted to achieve stable species, including both the reagents and the resulting complexes.

1.3 Results

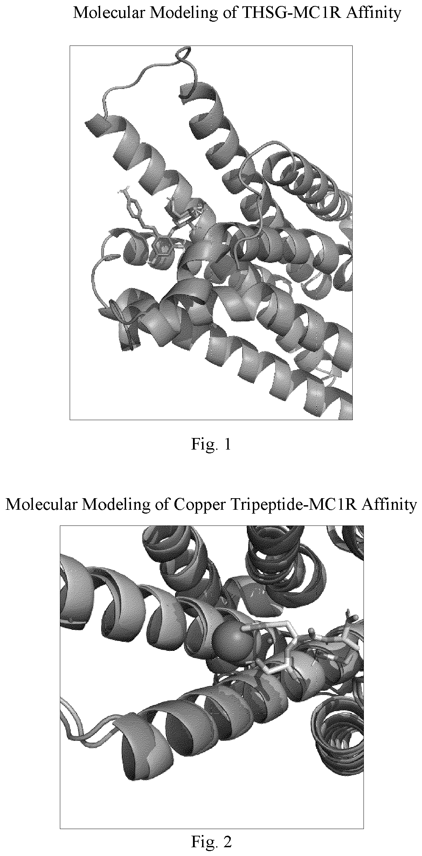

THSG was selected as the primary agent for the study. This substance has an estimated affinity score -7,5 kcal/mol (Figure 1), indicating a strong interaction.

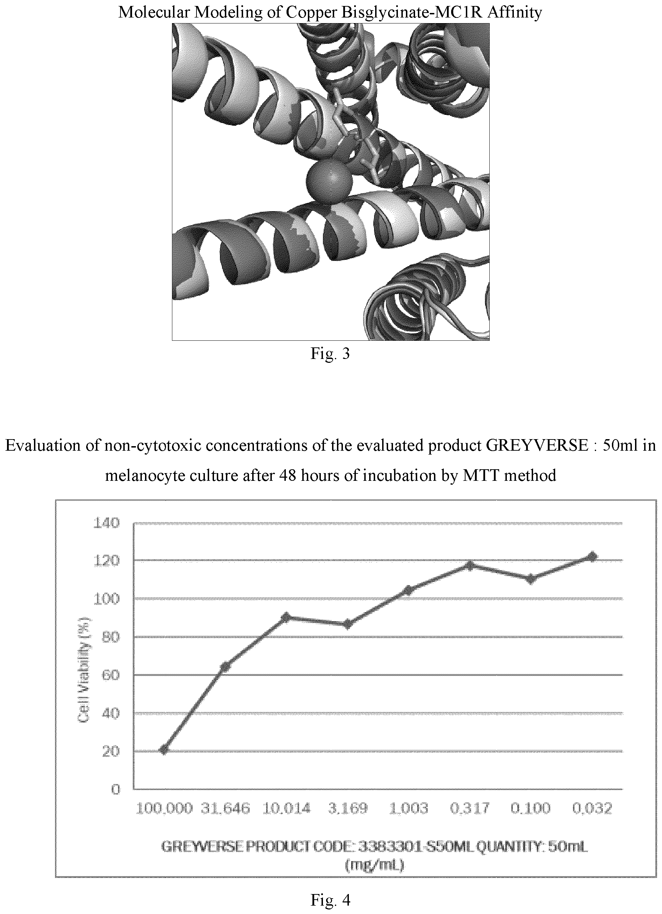

Copper tripeptide has an estimated affinity score -5,6 kcal/mol (Figure 2), Copper Bisglycinate has an estimated affinity score -4,3 kcal/mol (Figure 3). These metrics indicate a strong interaction between Copper Compounds and MC1R.

Conclusion: The affinity of THSG and Copper compounds with MC1R protein, confirmed using artificial intelligence, shows a potential to enhance melanin production. These findings suggest that THSG and Copper Compound could be a focus for future research, with a view to confirm in-vitro these properties.

**Example 2.** In-vitro cytotoxicity study of the investigated compounds: THSG and Copper Tripeptide, and market benchmark Greyverse

2.1 Materials and methods

2.1.1 Investigated ingredients

Table 2. Investigated ingredients

| No. | Ingredient |
|-----|------------|
| 1 | 2,3,5,4' - Tetrahydroxystilbene 2-O-$\beta$-D-Glucoside |
| 2 | Copper Tripeptide |
| 3 | Greyverse |

2.2 Investigation

Human melanocytes were seeded in 75 cm$^2$ flasks (Corning, USA), cultured in a specific culture medium, and expanded in an incubator at 37°C in the presence of 5% CO2. Upon reaching confluency, the cells were seeded in 6-well plates (Corning, USA) for subsequent quantification of the proposed mediator.

2.2.1 Evaluation of non-cytotoxic concentrations of THSG and Greyverse by MTT in melanocyte culture

Cell viability was determined by a colorimetric method using MTT dye (Sigma Chemical, St. Louis, Mo.). To perform the test, the products were prepared in culture medium and applied to a 96-well plate in a serial dilution in the range of 100 to 0.03 mg/mL using a dilution factor of 3.168. Human melanocytes were seeded in 75 cm$^2$ flasks (Corning, USA), cultured in a specific culture medium, and expanded in an incubator at 37°C in the presence of 5% CO2. Upon reaching confluency, the cells were seeded in 96- and 6-well plates (Corning, USA) for the determination of non-cytotoxic concentrations of the evaluated products. The melanocytes culture was incubated for a period of 48 hours. MTT was then added to the culture at a concentration of 0.5 mg/ml (100 $\mu$l/well) and incubated for an additional 4 hours. The contents of the well were removed and 100 $\mu$L of isopropanol was added to solubilize the formazan crystals formed. The absorbance of each well was determined at 570 nm on a Multiskan GO monochromator (Thermo Scientific, Finland). Cell viability was expressed as a percentage and calculated according to the equation:

$$\% \text{ Cell viability} = \frac{\text{Absorbance of cells incubated with the product}}{\text{Control absorbance} \times 100}$$

2.2.2 Evaluation of non-cytotoxic concentrations of Copper Tripeptide and Copper Bisglucinate by MTT in fibroblast culture

Cytotoxicity of substances was assessed using cell lines of non-immortalised fibroblasts in the MTT metabolic test. This method is based on the reduction reaction of the yellow tetrazolium salt (MTT) by mitochondrial dehydrogenases of living cells to purple formazan crystals, which are insoluble in aqueous medium.

For the assay, cells were cultured in DMEM medium supplemented with 20% fetal calf serum, L-glutamine and 1% penicillin/streptomycin in an atmosphere of 5% $CO_2$ at 37°C until monolayer formation. To perform the test, 1000÷3000 cells in 200 microlitres of culture medium are added to the wells of a 96-well plate depending on the proliferative potential. Cells are thoroughly suspended before introduction into the plate. The cell suspension was introduced into the wells of the plate using a multichannel pipette and sterile tips. The plates with cells were then incubated for 24 hours in a $CO_2$ incubator for cell adhesion to the substrate. Using a multichannel pipette and sterile tips, aliquots of prepared solutions of compounds at the tested concentrations were added to the wells of the plate with cells. After addition of the test substances, the cells were cultured in a $CO_2$ incubator under standard conditions for 48 hours. At the end of the incubation time, the culture medium with the test substances was removed from the plate. A mixture was prepared in a multi-channel pipetting tub: 9 ml of culture medium + 1 ml of MTT reagent (5 mg/ml in Hanks' solution) per 96-well plate. The prepared reaction mixture was added in a volume of 100 $\mu$l into each well of the plate and incubated in a $CO_2$ incubator for 3.5 hours.

At the end of the incubation time, the culture medium with the tested compounds was removed, 100 $\mu$l of DMSO was added and incubated for 5-10 minutes. The resulting violet staining was detected at a wavelength of 550 nm on a Tecan infinite 200 Pro micro-plate reader (Switzerland).

The ability of Copper Tripeptide to exert cytotoxic effect on cells was also evaluated by microscopy with counting the area of viable cells in the programme 'BCAn-alyzer' on fibroblast cell lines.

2.3 Results

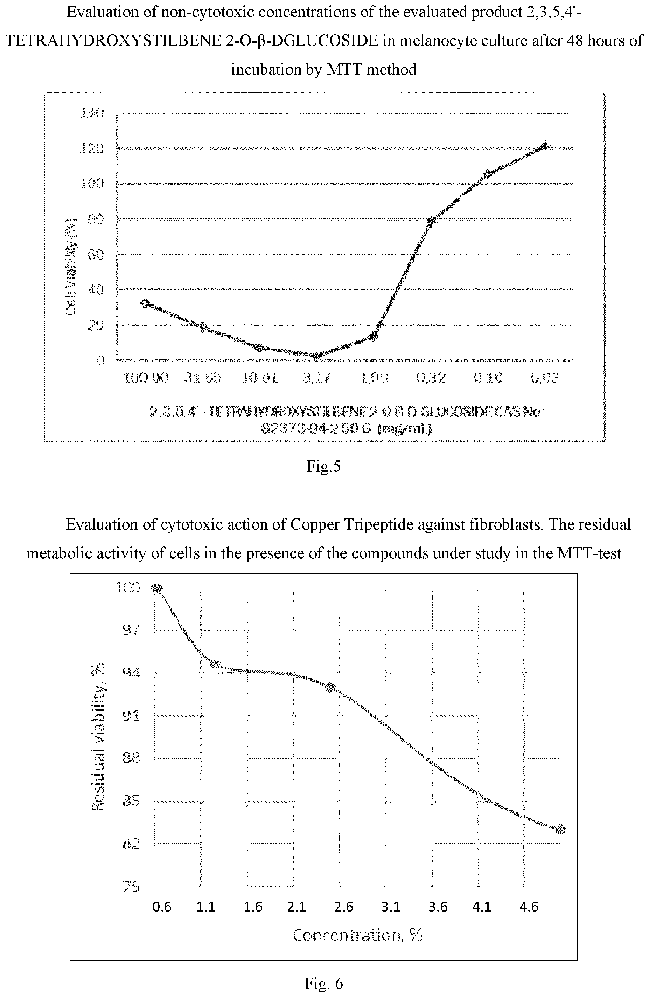

The cytotoxicity of THSG and Greyverse against non-immortalised melanocyte cell line was investigated at a concentration range of 0.032% to 100%. Figures 4 and 5 illustrate the residual metabolic activity of the cells in the presence of the tested compound as a result of the MTT assay.

As seen in Figure 4, the evaluated product GREYVERSE PRODUCT CODE: 3383301-S50ML showed non-cytotoxic concentrations when starting the dilution of 10.00 mg/mL.

The cytotoxicity of Copper Tripeptide against non-immortalised fibroblast cell line was investigated at a concentration range of 0.6% to 5%. Figure 6 illustrates the residual metabolic activity of the cells in the presence of the tested compound as a result of the MTT assay. For fibroblasts, the $IC_{50}$ for Copper Tripeptide was significantly found to be 28.78%. The value was obtained by extrapolation method.

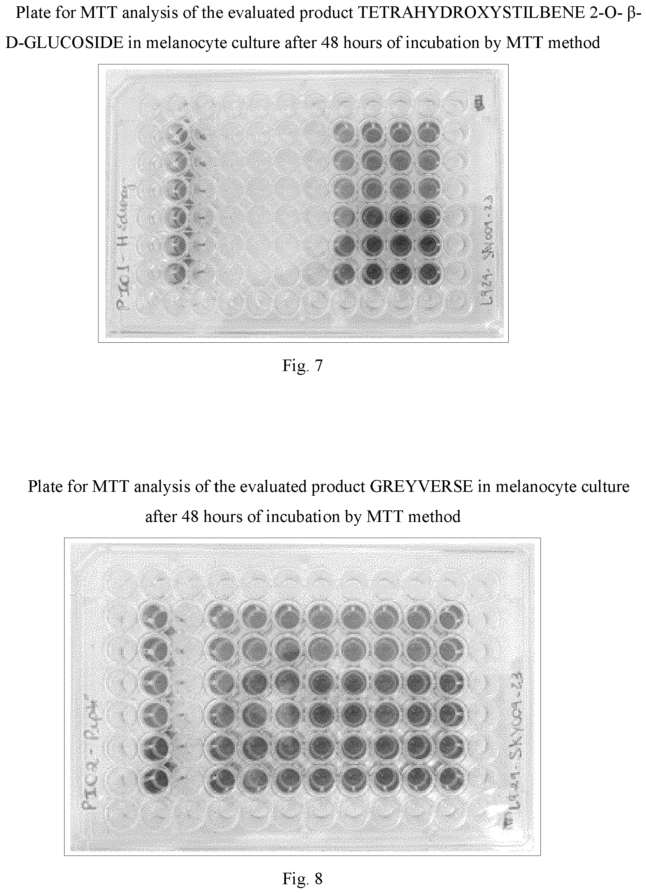

Figures 7 and 8 show visual differences in the effects of the actives on melanocytes after 48h incubation.

Conclusion: The results of non-cytotoxic concentration studies conducted on melanocyte and fibroblast cell lines identify a range of component performance that does not harm or reduce the efficiency of melanin production (due to reduced cell counts). Thus, the non-cytotoxic concentrations of THSG and Copper Compounds are 0 to 0.2% and 0 to 1%, respectively. Non-cytotoxic concentrations of Greyvers are 0 to 1%.

**Example 3.** Determination of antioxidant activity towards ascorbic acid for a liquid sample using the Ferric reducing antioxidant power (FRAP)

3.1 Materials and methods

3.1.1 Investigated ingredients

Table 3. Investigated ingredients in the FRAP assay

| No. | Ingredient |
|-----|------------|
| 1 | 2,3,5,4' - Tetrahydroxystilbene 2-O-β-D-Glucoside |
| 2 | Copper Tripeptide |
| 3 | Copper Bisglycinate |

For the experiment, 0.16% (w/v) aqueous solutions of water-soluble THSG, 0.005% (w/v) aqueous solutions of water-soluble Copper Tripeptide and 0.1% (w/v) aqueous solutions of water-soluble Copper Bisglucinate were prepared. Each compound, weighing 0.16 g, was dissolved in 100 mL of distilled or deionized water, with the aid of a magnetic stirrer to ensure complete dissolution.

3.2 Investigation

The FRAP assay was conducted in alignment with a modified version of the protocol originally delineated by Benzie and Strain (1996). All utilized reagents were of analytical grade and procured from recognized suppliers to ensure the assay's consistency and reliability. A 300 mM acetate buffer at pH 3.6 was prepared by dissolving 3.1 g of sodium acetate trihydrate (Sigma-Aldrich, St. Louis, MO, USA) in 16 mL of glacial acetic acid (Fisher Scientific, Hampton, NH, USA), with the volume adjusted to 1 L using distilled water. The TPTZ (2,4,6-tripyridyl-s-triazine) solution was made at 10 mM concentration in 40 mM HCl, and a 20 mM solution of $FeCl_3 \cdot 6H_2O$ was also prepared, both sourced from Sigma-Aldrich (St. Louis, MO, USA). For the preparation of the FRAP reagent, acetate buffer, TPTZ solution, and $FeCl_3 \cdot 6H_2O$ solution were combined in a 10:1:1 (v/v/v) ratio. This mixture was subsequently warmed to 37°C in a water bath (Julabo SW23, Julabo GmbH, Seelbach, Germany) immediately prior to the assay to ensure the reaction proceeded under optimal conditions. In the assay, 150 μL of the FRAP reagent was dispensed into each well of a 96-well plate, to which 50 μL of either the prepared sample or ascorbic acid standard was added. The components were thoroughly mixed using a vortex mixer (Vortex-Genie 2, Scientific Industries, Bohemia, NY, USA) to ensure uniformity.

The reaction mixtures were then incubated at 37°C for 30 minutes in a dark environment, allowing the reduction reactions to complete. Post incubation, the absorbance of each mixture was determined at 593 nm using a spectrophotometer (Shimadzu UV-1800, Shimadzu Corporation, Kyoto, Japan).

Absorbance values obtained from blank samples were subtracted from all other readings to correct for any baseline absorbance. The antioxidant capacities of the samples were quantified by comparing their absorbance values to those of an ascorbic acid standard curve, which was plotted under identical conditions.

This procedure was meticulously outlined to detail the execution of the FRAP assay, ensuring the reproducibility and reliability of the measurements of antioxidant capacities in the analyzed samples.

The general formula used to calculate the antioxidant capacity of the samples in the assay is as follows:

$$\text{Antioxidant Capacity} = \frac{(\text{Absorbance of Standart} - \text{Absorbance of Blank})}{(\text{Assorbance of Sample} - \text{Absorbance of Blank})} \times \text{Concentration of Standart}$$

Absorbance of Sample is the measured absorbance of the reaction mixture containing the test sample. Absorbance of Blank is the absorbance of the reaction mixture containing all reagents except the sample or standard (negative control). Absorbance of Standard is the measured absorbance of the reaction mixture containing a known concentration of the standard antioxidant (ascorbic acid). Concentration of Standard is the known concentration of ascorbic acid used in the assay.

3.3 Result

Table 4. Results of the Antioxidant Capacity for investigated ingredients

| Ingredient | Antioxidant Capacity (Relative to Vitamin C), % |
|---|---|
| 2,3,5,4' - Tetrahydroxystilbene 2-O-B-D-Glucoside (0.16%) | 37.31 (p < 0.05) |
| Copper Tripeptide (0.005%) | -0.40 (p < 0.05) |
| Copper Bisglucinate (0.1%) | 1.24 (p < 0.05) |
| 2,3,5,4' - Tetrahydroxystilbene 2-O-B-D-Glucoside (0.16%) + Copper Tripeptide (0.005%) | 48.86 (p < 0.05) |
| 2,3,5,4' - Tetrahydroxystilbene 2-O-B-D-Glucoside (0.16%) + Copper Bisglycinate (0.1%) | 43.21 (p < 0.05) |

In this study, THSG, Copper Tripeptide and Copper Bisglycinate were evaluated for their antioxidant capacities against Ascorbic acid (100%). Notably, THSG emerged as the standout, exhibiting the highest antioxidant capacity at 37.31% relative to Vitamin C. This highlights its potential as a superior antioxidant. In combination with Copper Tripeptide, there is a synergy resulting in an antioxidant activity enhanced by 30.96% relative to the activity of solo THSG. Thus, the use of combination of THSG and Copper Tripeptide looks promising for use as an anti-grey hair product, as it solves the oxidative stress issue.

**Example 4.** Study of the effect of THSG, Copper Tripeptide, Copper Bisglycinate, GREYVERSE on melanin production in human melanocyte culture

4.1 Materials and methods

4.1.1 Investigated ingredients

Table 5. Investigated ingredients in-silico study

| No. | Ingredient |
|---|---|
| 1 | 2,3,5,4' - Tetrahydroxystilbene 2-O-B-D-Glucoside |
| 2 | Copper Tripeptide |
| 3 | Copper Bisglycinate |
| 4 | Greyverse |

4.2 Investigation

Human melanocytes were seeded in 75 cm$^2$ flasks (Corning, USA), cultured in a specific culture medium, and expanded in an incubator at 37°C in the presence of 5% CO2. Upon reaching confluency, the cells were seeded in 6-well plates (Corning, USA) for subsequent quantification of the proposed mediator.

4.2.1 Evaluation of the effects of THSG and Greyverse on melanin production

Evaluation of the preclinical effects of three previously determined non-cytotoxic concentrations of the evaluated product GREYVERSE PRODUCT CODE: 3383301-S50ML and 2,3,5,4' - tetrahydroxystilbene 2-O-B-D-glucoside, on melanin production in culture of human melanocytes.
Melanin concentration was measured in a 405 nm spectrophotometer in the cell lysate of melanocyte cultures and compared based on a curve with concentrations of 500.00-1.95 $\mu$g/mL melanin (Sigma).
The statistical evaluation used the ANOVA test to measure the variation of the results, comparing the data between the groups. The Bonferroni posttest was then applied, which strengthened and made the result presented in the ANOVA more precise. The 5% significance level was used.

4.2.2 Evaluation of the effects of THSG, Copper Compounds on melanin production

The cell cultures were incubated with 3 non-cytotoxic concentrations of the evaluated product Copper Compounds added with 0.13% of the evaluated product 2,3,5,4' - tetrahydroxystilbene 2-O-$\beta$-D-glucoside CAS No: 82373-94-2. The concentrations of the evaluated product Copper Compounds were 0,01, 0.005 and 0.001 mg/ml. For quantifications of melanin synthesis, human melanocytes were treated with the products for 48 hours.
Melanin concentration was measured in a 405 nm spectrophotometer in the cell lysate of melanocyte cultures and compared based on a curve with concentrations of 500.00-1.95 $\mu$g/mL melanin (Sigma).
The statistical evaluation used the ANOVA test to measure the variation of the results, comparing the data between the groups. The Bonferroni posttest was then applied, which strengthened and made the result presented in the ANOVA more precise. The 5% significance level was used.

4.3 Results

Table 6. Percentage increase in melanin production when using Greyverse

| No. | Concentration, % | Increase in melanin production, % |
|---|---|---|
| 1 | 2.0 | 28.54 |
| 2 | 1.0 | 24.23 |
| 3 | 0.5 | 21.44 |

Table 7. Percentage increase in melanin production when using 2,3,5,4' - tetrahydroxystilbene 2-O- β-D-glucoside

| No. | Concentration, % | Increase in melanin production, % |
|---|---|---|
| 1 | 0.3 | 31.67 |
| 2 | 0.2 | 24.59 |
| 3 | 0.1 | 22.90 |

Table 8. Percentage increase in melanin production when using 0.13% 2,3,5,4' - tetrahydroxystilbene 2-O- β-D-glucoside and Copper Compounds

| No. | Concentration of Copper Compound, % | Increase in melanin production, % |
|---|---|---|
| 1 | 0.01 | 36.19 |
| 2 | 0.005 | 30.06 |
| 3 | 0.001 | 25.15 |

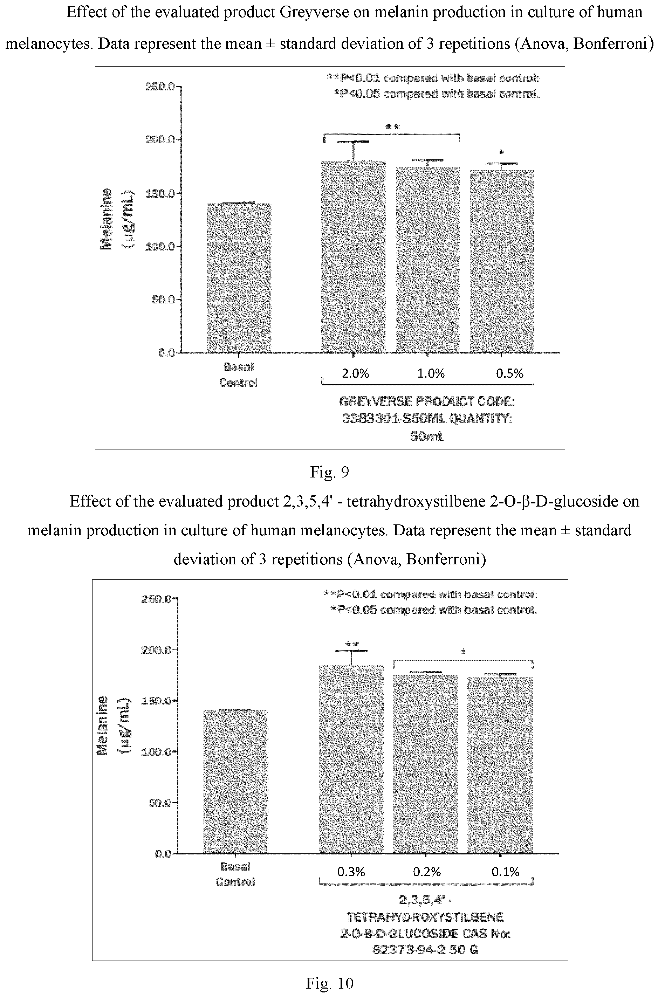

Figure 9 represents the total melanin production obtained in the melanocyte cultures treated with Greyverse. Figure 10 represents the total melanin production obtained in the melanocyte cultures treated with 2,3,5,4' - tetrahydroxystilbene 2-O- β-D-glucoside. Figure 11 represents the total melanin production obtained in the melanocyte cultures treated with the evaluated products Copper Compounds and 2,3,5,4' - tetrahydroxystilbene 2-O- β-D-glucoside. Culture's treatment demonstrated a significant modulation on melanin synthesis in 36.19%($p < 0.01$), 30.06%($p < 0.05$) and 25.15% ($p < 0.05$), respectively, at the evaluated concentrations 0.01, 0.005 and 0.001 mg/ml of the evaluated product Copper Compound added with 0.13% of 2,3,5,4' - tetrahydroxystilbene 2-O-β-D-glucoside each, in relation to the basal control.
The results obtained allow us to infer that the evaluated product Copper Compound added with 0.13% of the evaluated product 2,3,5,4' - tetrahydroxystilbene 2-O-β-D-glucoside increases the synthesis of melanin, therefore, we can infer that the evaluated products Copper Compound and 2,3,5,4' - tetrahydroxystilbene 2-O-β-D-glucoside product have pigmentation activity.
Water-soluble copper compounds can serve as effective copper sources. These compounds include, but are not limited to, copper tripeptide, copper bisglycinate, copper lysinate/prolinate, Saccharomyces copper enzyme, copper picolinate, copper glycinate, copper aspartate, and copper gluconate.

**Example 5.** Evaluation of antiseptic efficacy using the time kill test for *Malassezia furfur*

5.1 Materials and methods

5.1.2 Investigated ingredients

Table 9. Investigated ingredients

| No. | Ingredient |
|---|---|
| 1 | 2,3,5,4' - Tetrahydroxystilbene 2-O-B-D-Glucoside |

5.2 Investigation

The microorganism *Malassezia furfur* (ATCC 14521) was inoculated into the investigational product. Consecutive subcultures of *Malassezia furfur* (ATCC 14521) were grown in specific broth and incubated at 30°C for 24 hours. The third subculture was quantified according to Pharmacopoeial parameters (106 CFU/mL). After determining the microbial load, the microorganisms were inoculated on the product evaluated. The inoculum was diluted at the ratio of 1:100 for the test. For this test, the investigational product 2,3,5,4' - Tetrahydroxystilbene 2-O- β-D-Glucoside was diluted in sterile deionized water at 0.13%.

The suspension of the microorganism was inoculated into the sample. Aliquots of the suspension were collected after 30 seconds, 1 minute and 3 minutes of contact. They were then neutralized and diluted in 0.1% peptone water, then plated on the appropriate culture medium. Incubation was carried out for 72 hours at 30°C. At the end of this period, the colonies present were evaluated and measured.

The evaluated product was subjected to a sanitizing activity investigation using the linear regression method. To determine the lethality profile (log CFU/mL vs. time), the ability of the product to reduce, on a logarithmic scale, the standardized microbial populations after 30 seconds, 1 minute and 3 minutes of contact was verified.

5.3 Results

Table 10. Results obtained with group 1 - 2,3,5,4' - Tetrahydroxystilbene 2-O-β-D-Glucoside - 0,13% at the corresponding contact times

| Evaluated microorganism | Malassezia furfur (ATCC 14521) | | |
|---|---|---|---|
| Initial population | Contact time | | |
| | 30 seconds | 1 minute | 3 minutes |
| $2.0\times10^4$ (CFU/mL) | $2.4\times10^4$ (CFU/mL) | $1.4\times10^4$ (CFU/mL) | $6.0\times10^3$ (CFU/mL) |
| Logarithmic Reduction | 0.0 | 0.15 | 0.52 |
| Percentage of microbial reduction | 0.00% | 30.00% | 70.00% |

The products 2,3,5,4' - Tetrahydroxystilbene 2-O-β-D-Glucoside showed antimicrobial and antiseptic activity. Specifically for the strain *Malassezia furfur* (ATCC 14521), it produced a 70% reduction after 3 minutes of contact with the microorganism.

**Example 6.** Stability studies of components at different temperatures

6.1 Materials and methods

6.1.1 Investigated solution

Table 11. Investigated solution

| Compounds | Concentration |
|---|---|
| 2,3,5,4' - Tetrahydroxystilbene 2-O-B-D-Glucoside | 0.1% |

The solution was prepared in distilled water. The pH was adjusted to 6.5. The solutions were stored for stability evaluation under the following conditions: at 40°C, 25°C, 4°C.

The solutions were stored for 40 days, then solutions which were kept at 40°C and 25°C were sent for component composition study.

6.2 Investigation

Stability results after 40 days were evaluated for the following parameters: pH, appearance, and color.

The analysis to determine the composition was carried out by ultra-performance liquid chromatography/MS on a Waters Acquity UPLC chromatograph with diode matrix UV detector and TQD tandem quadrupole MC detector (Waters).

Mobile phase A (MP A). Water-acetonitrile (95:5) mixture with formic acid.

Mobile phase B (MP B). Acetonitrile with formic acid.

The test solutions were chromatographed under the following conditions:

- 2 μl sample volume;

- 0.21 × 5.0 cm Acquity UPLC BEH C18 column (1.7 pm);

- column temperature 35°C;

- flow rate 0.5 ml/min;

- gradient chromatography mode is formed by mixing mobile phases A and B according to the scheme in the Table 12.

Table 12. Scheme for mixing the mobile phases

| Time, min | MP A, % | MP B, % |
|---|---|---|
| 0 | 100 | 0 |
| 4.0 | 0 | 100 |
| 4.1 | 100 | 0 |
| 4.0 | 100 | 0 |

UV detection: 220-500 nm.
MS conditions:

1) MS detection in negative ion mode:

- detector parameters: capillary voltage - 3 kV; cone voltage
- 31 V; capillary temperature 450 0C; source temperature 120 0C; drying gas flow rate 800 l/h, cone gas flow rate 50 l/h and scanning in the mass range from 80 to 1000 units;

2) MS detection in positive ion mode:

- detector parameters: capillary voltage - 3 kV; cone voltage
- 50 V; capillary temperature 450 0C; source temperature 120 0C; drying gas flow rate 800 l/h, gas flow rate in the cone 50 l/h and scanning in the mass range from 80 to 1000 units.

6.3 Results

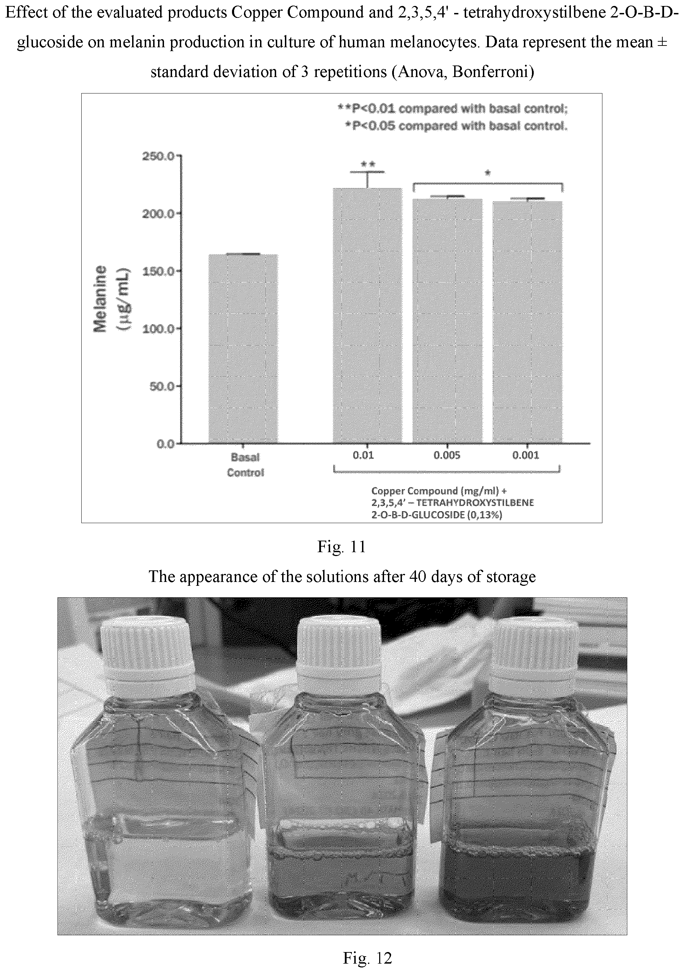

The appearance of the solutions after 40 days is shown in Figure 12. Table 13 summarizes the results of the analysis of these solutions.

Table 13. Stability results

| Temperature of stora ge, °C | pH | | | Color | | | Appearance | | |
|---|---|---|---|---|---|---|---|---|---|
| | 0 days | 14 days | 40 days | 0 days | 14 days | 40 days | 0 days | 14 days | 40 days |
| 40 | | 6.31 | 6.55 | | light brown | light brown | | | |
| 25 | 6.7 | 6.65 | 6.64 | Transp arent | light yellow | yellow | homogeneous clear liquid | | |
| 4 | | 6.67 | 6.63 | | very light yellow | light yellow | | | |

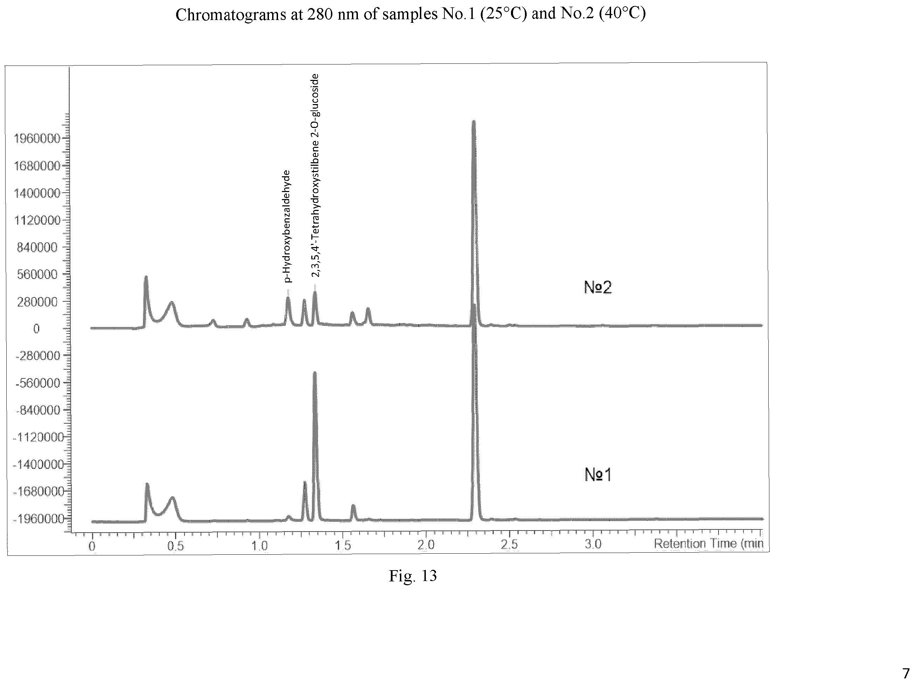

Solutions that were stored at temperatures of 40°C and 25°C darkened. We carried out a component study to identify what this was due to.
The following chromatograms at 280 nm of samples No.1 (25°C) and No.2 (40°C) were obtained by component analysis. The results are shown in Figure 13.

The results demonstrate that after degradation at 40°C, new components were formed, and the concentration of previously detected components changed. The values are presented in Table 14.

Table 14. Ratio of concentration and availability of components in solutions stored at 40°C and 25°C.

| Name | $t_R$ | Peak Area (280 nm) | | Change in concentration of components in relation to sample No.1 |
|---|---|---|---|---|
| | | 40°C | 25°C | |
| p-Hydroxybenzaldehy de | 1.18 | 3.9E+08 | 63646368 | +612.20% |
| 2,3,5,4' - Tetrahydroxystilb ene 2-O-β-D-Glucoside | 1.34 | 3.83E+0 8 | 1.76E+09 | -21.74% |

At 40°C, the concentration of 2,3,5,4'-Tetrahydroxystilbene 2-O-glucoside decreases by a factor of 0.22, while the concentration of p-Hydroxybenzaldehyde increases by a factor of 6.12. The observed increase in the color intensity of the solution is attributed to the formation of various oligomeric degradation products that absorb light in the visible spectrum.

Based on the findings of this study, it can be concluded that the application of 2,3,5,4'-Tetrahydroxystilbene 2-O-β-D-Glucoside is restricted to a temperature range not exceeding 25°C, as degradation of the molecule begins at higher temperatures.

REFERENCES

1. PanhardS, Lozano I, LoussouarnG. Greying of the human hair: a worldwide survey, revisiting the '50' rule of thumb. Br J Dermatol. 2012 Oct; 167(4) :865-73.

2. ParkAM, KhanS, RawnsleyJ. Hair Biology: growth and Pigmentation. Facial Plast Surg Clin North Am. 2018 Nov;26(4):415-24.

3. KumarAB, ShamimH, NagarajuU. Premature Graying of Hair: review with Updates. Int J Trichology. 2018 Sep-Oct;10(5):198-203.

4. TobinD. (2010). The Aging Hair Pigmentary Unit. In: R. Trueb and D. Tobin, ed., Aging Hair, 1st ed. Verlag Berlin Heidelberg: Springer, pp.77-89

5. Tobin DJ, PausR. Graying: gerontobiology of the hair follicle pigmentary unit. Exp Gerontol. 2001Jan;36(1) :29-54.

6. Jo SJ, PaikSH, Choi JW, Lee JH, ChoS, KimKH, et al Hair graying pattern depends on gender, onset age and smoking habits. Acta Derm Venereol. 2012 Mar; 92(2) :160-1.

7. SatoS, JitsukawaK, SatoH, YoshinoM, SetaS, ItoS, et alSegmented heterochromia in black scalp hair associated with iron-deficiency anemia. Canities segmentata sideropaenica. Arch Dermatol. 1989Apr;125(4) :531-5.

8. NoppakunN, SwasdikulD. Reversible hyperpigmentation of skin and nails with white hair due to vitamin B12 deficiency. Arch Dermatol. 1986Aug;122 (8) :896-9.

9. AcerE, Kaya ErdoganH, IgrekA, ParlakH, SaracogluZ, BilginM. Relationship between diet, atopy, family history, and premature hair graying. J Cosmet Dermatol. 2018. [PubMed]1473-2130

10. Tobin DJ. The cell biology of human hair follicle pigmentation. Pigment Cell Melanoma Res. 2011Feb;24(1) :75-88.

11. StennKS, PausR. Controls of hair follicle cycling. Physiol Rev. 2001 Jan;81(1) :449-94

12. FitzpatrickTB, BrunetP, KukitaA. The nature of hair pigment. In: MontagnaW, EllisRA, editors. The Biology of Hair Growth. New York: Academic Press; 1958. pp. 255-303

13. ClineDJ. Changes in hair color. Dermatol Clin. 1988Apr;6(2):295-303.

14. ShaffraliFC, McDonaghAJ, MessengerAG. Hair darkening in porphyria cutanea tarda. Br J Dermatol. 2002Feb;146(2) :325-9.

15. PinkusH. Postinflammatory hair darkening. Arch Dermatol. 1960 Aug;82 (2) :263-4

16. VerbovJ. Erosive candidiasis of the scalp, followed by the reappearance of black hair after 40 years. Br J Dermatol. 1981 Nov;105(5) :595-8.

17. DalgicB, Egritas O. Gray hair and acrodermatitis enteropathica-like dermatitis: an unexpected presentation of cystic fibrosis. Eur J Pediatr. 2011 Oct;170(10):1305-8

18. Redondo P, Guzmán M, MarquinaM, PretelM, AguadoL, LloretP, et al [Repigmentation of gray hair after thyroid hormone treatment]. Actas Dermosifiliogr. 2007 Nov;98(9) :603-10

19. SeibergM. Age-induced hair greying - the multiple effects of oxidative stress. Int J Cosmet Sci. 2013Dec;35(6):532-8.

20. Wood JM, Decker H, HartmannH, ChavanB, Rokos H, Spencer JD, et alSenile hair graying: H2O2-mediated oxidative stress affects human hair color by blunting methionine sulfoxide repair. FASEB J. 2009 Jul;23(7) :2065-75.

21. Akin BelliA, EtguF, Ozbas GokS, KaraB, DoganG. Risk Factors for Premature Hair Graying in Young Turkish Adults. Pediatr Dermatol. 2016Jul;33(4) :438-42.

22. TrüebRM. Association between smoking and hair loss: another opportunity for health education against smoking?Dermatology. 2003;206(3) :189-91.

23. Niu C, Aisa HA. Upregulation of melanogenesis and tyrosinase activity: Potential agents for vitiligo. Molecules. 2017;22:1303-1305.

24. Rzepka Z, Buszman E, Beberok A, Wrześniok D. From tyrosine to melanin: Signaling pathways and factors regulating melanogenesis. Postepy Hig Med Dosw. 2016;70:695-708.

25. D'Ischia M, Wakamatsu K, Napolitano A, Briganti S, Garcia-Borron JC, Kovacs D, Meredith P, Pezzella A, Picardo M, Sarna T, et al. Melanins and melanogenesis: Methods, standards, protocols. Pigment Cell Melanoma Res. 2013;26:616-633.

26. Slominski A, Zmijewski MA, Pawelek J. L-tyrosine and L-di-hydroxyphenylalanine as hormone-like regulators of melanocyte functions. Pigment Cell Melanoma Res. 2012;25:14-27.

27. Pillaiyar T, Manickam M, Namasivayam V. Skin whitening agents: Medicinal chemistry perspective of tyrosinase inhibitors. J Enzyme Inhib Med Chem. 2017;32:403-425.

28. Hollinger JC, Angra K, Halder RM. Are natural ingredients effective in the management of hyperpigmentation? A systematic review. J Clin Aesthet Dermatol. 2018;11:28-37.

29. Pillaiyar T, Manickam M, Namasivayam V. Skin whitening agents: Medicinal chemistry perspective of tyrosinase inhibitors. J Enzyme Inhib Med Chem. 2017;32:403-425.

30. Caruntu C, Boda D, Constantin C, Caruntu A, Neagu M. Catecholamines increase in vitro proliferation of murine B16F10 melanoma cells. Acta Endocrinol (Copenh) 2014;10:545-558.

31. Diaconeasa A, Boda D, Solovan C, Enescu DM, Vilcea AM, Zurac S. Histopathologic features of Spitzoid lesions in different age groups. Rom J Morphol Embryol. 2013;54:51-62.

32. Hasegawa T. Tyrosinase-expressing neuronal cell line as in vitro model of Parkinson's disease. Int J Mol Sci. 2010;11:1082-1089. doi: 10.3390/ijms11031082.

33. Zurac S, Neagu M, Constantin C, Cioplea M, Nedelcu R, Bastian A, Popp C, Nichita L, Andrei R, Tebeica T, et al. Variations in the expression of TIMP1, TIMP2 and TIMP3 in cutaneous melanoma with regression and their possible function as prognostic predictors. Oncol Lett. 2016;11:3354-3360.

34. PavithranK. Puvasol Therapy in Premature Greying of Hair. Indian J Dermatol Venereol Leprol. 1986Mar-Apr;52(2) :74-5.

35. RobinsonA, Jones W. Changes in scalp hair after cancer chemotherapy. Eur J Cancer Clin Oncol. 1989Jan;25(1) :155-6.

36. Ward PD, Miller HL, Shipman AR. A case of repigmentation and curling of hair on acitretin therapy. Clin Exp Dermatol. 2014 Jan;39(1):91-2.

37. SeckinD, YildizA. Repigmentation and curling of hair after acitretin therapy. Australas J Dermatol. 2009Aug;50(3) :214-6.

38. Vesper JL, FenskeNA. Hair darkening and new growth associated with etretinate therapy. J Am Acad Dermatol. 1996May;34(5 Pt 1):860.

39. KavakA, AkcanY, KorkmazU. Hair repigmentation in a hepatitis C patient treated with interferon and ribavirin. Dermatology. 2005;211(2):171-2.

40. BabuKG, RasheshyamD, LalithaN. Canities-reversal with chemotherapy. J Assoc Physicians India. 1995Aug;43(8):577.[PubMed]0004-5772

41. LoveringS, MiaoW, BailieT, AmatoD. Hair repigmentation associated with thalidomide use for the treatment of multiple myeloma. BMJ Case Rep. 2016Jul;2016:bcr2016215521.

42. TintleSJ, DabadeTS, KalishRA, RosmarinDM. Repigmentation of hair following adalimumab therapy. Dermatol Online J. 2015Jun;21(6):13030/qt6fn0t1xz.[PubMed]1087-2108

43. DasanuCA, MitsisD, AlexandrescuDT. Hair repigmentation associated with the use of lenalidomide: graying may not be an irreversible process! J Oncol Pharm Pract. 2013Jun;19(2) :165-9.

44. KhaledA, TrojjetsS, ZeglaouiF, FazaaB, KamounMR. Repigmentation of the white hair after systemic corticosteroids for bullous pemphigoid. J Eur Acad Dermatol Venereol. 2008Aug;22 (8) :1018-20

45. SadighhaA, ZahedGM. Hair darkening after treatment with cyclosporin in a patient with psoriasis. J Eur Acad Dermatol Venereol. 2008Nov;22(10) :1239-41.

46. ReboraA, DelmonteS, ParodiA. Cyclosporin A-induced hair darkening. Int J Dermatol. 1999Mar;38(3):229-30.

47. EtienneG, Cony-Makhoul P, Mahon FX. Imatinib mesylate and gray hair. N Engl J Med. 2002Aug;347(6):446.

48. Cheng YP, ChenHJ, ChiuHC, ChenH. Erlotinib-induced hair repigmentation. Int J Dermatol. 2014Jan;53(1):e55-7.

49. AlexandrescuDT, Kauffman CL, Dasanu CA. Persistent hair growth during treatment with the EGFR inhibitor erlotinib. Dermatol Online J. 2009Mar;15(3):4

50. Bellandi S, AmatoL, CipolliniEM, AntigaE, Brandini L, FabbriP. Repigmentation of hair after latanoprost therapy. J Eur Acad Dermatol Venereol. 2011Dec;25(12):1485-7.

51. HampsonJP, DonnellyA, Lewis-JonesMS, PyeJK. Tamoxifen-induced hair colour change. Br J Dermatol. 1995Mar;132(3) :483-4.

52. ReynoldsNJ, CrossleyJ, FergusonI, PeacheyRD. Darkening of white hair in Parkinson's disease. Clin Exp Dermatol. 1989 Jul;14(4) :317-8.

53. Dizdar O. et al. Adjuvant use of epidermal growth factor receptor kinase inhibitors may prevent bone metastases in patients with non-small cell lung carcinoma //Journal of Thoracic Oncology. - 2007. - T. 2. - No. 12. - C. 1136.

54. Grainger KM. Pigmentation in Parkinson's disease treated with levodopa. Lancet. 1973Jan;1(7794):97-8.

55. Pasricha J. Successful treatment of gray hairs wtih high dose calcium pantothenate. Indian J Dermatol Venereol Leprol. 1981;47(6):311-3.0378-6323

56. PasrichaJS. Effect of Grey Hair Evulsion on the response to calcium pantothenate in premature grey hairs. Indian J Dermatol Venereol Leprol. 1986Mar-Apr;52(2):77-80.[PubMed]0973-3922

57. Sieve B. Clinical Achromotrichia. Science (80-) . 1941;94(2437):257-258

58. ZarafonetisCJ. Darkening of gray hair during para-aminobenzoic acid therapy. J Invest Dermatol. 1950Dec;15(6):399-401.

59. Brandaleone H, MainE, SteeleJ. The effect of calcium pantothenate and para-aminobenzoic acid on gray hair. Exp Biol Med. 1943;53(1):47-9.

60. PhilipM, Samson JF, SimiPS. Clofazimine-induced Hair Pigmentation. Int J Trichology. 2012Jul;4(3):174-5.

61. Read GM. Verapamil and hair colour change. Lancet. 1991 Dec; 338 (8781) :1520.

62. Drug Information. UpToDate. www.uptodate.com. Published 2019.

63. ChristophT, Müller-RöverS, AudringH, Tobin DJ, HermesB, Cotsarelis G, et alThe human hair follicle immune system: cellular composition and immune privilege. Br J Dermatol. 2000May;142 (5) :862-73.

64. BsYM, DiehlJ, LevinsPC. Promising alternative clinical uses of prostaglandin F2$\alpha$ analogs: beyond the eyelashes. J Am Dermatology. 2015;72 (4) :712-6.

65. Chavan D. Reversal of Premature Hair Graying Treated with a Topical Formulation Containing $\alpha$-Melanocyte-Stimulating Hormone Agonist (Greyverse Solution 2%) //International Journal of Trichology. - 2022. - T. 14. - No.6. - C. 207-209.

66. Patent WO2020089216A1. Hair care active agent. URL: https://patents.google.com/patent/WO2020089216A1/en

67. Zequn Jiang, Jimin Xu, Minghai Long, Zhiming Tu, Guangxiao Yang, Guangyuan He. 2, 3, 5, 4'-tetrahydroxystilbene-2-O-$\beta$-D-glucoside (THSG) induces melanogenesis in B16 cells by MAP kinase activation and tyrosinase upregulation. Life Sciences 85 (2009) 345-350.

68. LiverTox: Clinical and Research Information on Drug-Induced Liver Injury [Internet]. Bethesda (MD): National Institute of Diabetes and Digestive and Kidney Diseases; 2012-. Polygonum Multiflorum. [Updated 2020 Aug 18]. Available from: https://www.ncbi.nlm.nih.gov/books/NBK548795/

69. Dong X, Fu J, Yin X, Cao S, Li X, Lin L; Huyiligeqi; Ni J. Emodin: A Review of its Pharmacology, Toxicity and Pharmacokinetics. Phytother Res. 2016 Aug;30(8):1207-18. doi: 10.1002/ptr.5631. Epub 2016 May 18. PMID: 27188216; PMCID: PMC7168079.

70. Pyo HK, Yoo HG, Won CH, Lee SH, Kang YJ, Eun HC, Cho KH, Kim KH. The effect of tripeptide-copper complex on human hair growth in vitro. Arch Pharm Res. 2007 Jul;30(7):834-9. doi: 10.1007/BF02978833. PMID: 17703734

71. Millington KR, Marsh JM. UV damage to hair and the effect of antioxidants and metal chelators. Int J Cosmet Sci. 2020 Apr;42(2):174-184. doi: 10.1111/ics.12601. Epub 2020 Mar 11. PMID: 31955440

72. Patent KR20120048830A. A method for preparing the treatment with tripeptide derivatives for prematurely gray hair. URL: https://patents.google.com/patent/KR20120048830A/en

- 73. Kim S C.J., Cheng T, Gindulyte A, He J, He S, Li Q, Shoemaker BA, Thiessen PA, Yu B, Zaslavsky L, Zhang J, Bolton EE. PubChem 2023 update. // Nucleic Acids Res.. 2023. V. 6. No. 51(D1). P. 1373.

- 74. Neese F. Software update: The ORCA program system-Version 5.0 // WIREs Computational Molecular Science. 2022. V. 12. No.5. P. e1606.

- 75. Adamo C., Barone V. Toward reliable density functional methods without adjustable parameters: The PBE0 model // The Journal of Chemical Physics. 1999. V. 110. No.13. P. 6158.

- 76. Grimme S., Ehrlich S., Goerigk L. Effect of the damping function in dispersion corrected density functional theory // Journal of Computational Chemistry. 2011. V. 32. No. 7. P. 1456.

- 77. Weigend F., Ahlrichs R. Balanced basis sets of split valence, triple zeta valence and quadruple zeta valence quality for H to Rn: Design and assessment of accuracy // Physical Chemistry Chemical Physics. 2005. V. 7. No.18. P. 3297.

## Claims

1. A composition comprising the following components A and B:

   A) 2,3,5,4'-Tetrahydroxystilbene 2-O-β-D-glucoside, preferably less than 0.2% by weight of said composition, and
   B) at least one compound comprising copper, preferably less than 1% by weight of said composition.

2. The composition of claim 1, wherein said component B is selected from the group consisting of copper tripeptide, copper bisglycinate, copper lysinate and/or prolinate, *Saccharomyces* copper enzyme, copper picolinate, copper glycinate, copper aspartate, and copper gluconate, or any combination thereof.

3. The composition of claim 2, wherein said component B is copper bisglycinate alone or in combination with copper tripeptide.

4. The composition of claim 2, wherein said component B is copper tripeptide alone or in combination with copper bisglycinate.

5. The composition of any of claims 2-4, wherein said copper tripeptide comprises a positively charged copper ion and amino acid residues L-alanyl-L-histidyl-L-lysine.

6. The composition of any of the preceding claims,
   wherein said composition is assembled as single composition comprising said components A and B, or as a kit-of-parts composition separating said components A and B into separate entities, and wherein said separated entity parts of said kit-of-parts are for combining said components A and B prior use.

7. The composition of any of the preceding claims,
   wherein said composition is formulated for application on a subject's hair and/or scalp.

8. The composition of any of the preceding claims,
   wherein said composition is a formulation selected from the following: liquid, aerosol, suspension, solution, tincture, cream, paste, lotion, ointment, gel, balsam, foam, shampoo.

9. The composition of any of the preceding claims,
   wherein said composition is for preventing and/or reversing hair greying and/or dandruff in a subject, wherein said subject is preferably a human or an animal.

10. Use of the composition of any of the preceding claims in the non-medical treatment of the scalp and or hair of a subject, including cosmetic, aesthetic and dermatological non-medical treatment.

11. The use of claim 10, wherein said non-medical treatment prevents and/or reverses hair greying and/or dandruff in a subject, wherein said subject is preferably a human or animal.

12. The composition of any of claims 1-8 for use as a medicament.

13. The composition for use of claim 11, wherein said medicament is selected from the group consisting of an antiseptic, antimicrobial and antioxidant.

14. The composition of any of claims 1-8 for use in the treatment of a disease selected from the group consisting of premature graying of hair (PGH), achromotrichia, canities and hypomelanosis.

15. The use of 2,3,5,4'-Tetrahydroxystilbene 2-O-$\beta$-D-glucoside in combination with at least one compound comprising copper in the manufacture of composition of any of claims 1-7.

Molecular Modeling of THSG-MC1R Affinity

Fig. 1

Molecular Modeling of Copper Tripeptide-MC1R Affinity

Fig. 2

Molecular Modeling of Copper Bisglycinate-MC1R Affinity

Fig. 3

Evaluation of non-cytotoxic concentrations of the evaluated product GREYVERSE : 50ml in melanocyte culture after 48 hours of incubation by MTT method

Fig. 4

Evaluation of non-cytotoxic concentrations of the evaluated product 2,3,5,4'-TETRAHYDROXYSTILBENE 2-O-β-DGLUCOSIDE in melanocyte culture after 48 hours of incubation by MTT method

Fig.5

Evaluation of cytotoxic action of Copper Tripeptide against fibroblasts. The residual metabolic activity of cells in the presence of the compounds under study in the MTT-test

Fig. 6

Plate for MTT analysis of the evaluated product TETRAHYDROXYSTILBENE 2-O- β-
D-GLUCOSIDE in melanocyte culture after 48 hours of incubation by MTT method

Fig. 7

Plate for MTT analysis of the evaluated product GREYVERSE in melanocyte culture
after 48 hours of incubation by MTT method

Fig. 8

Effect of the evaluated product Greyverse on melanin production in culture of human melanocytes. Data represent the mean ± standard deviation of 3 repetitions (Anova, Bonferroni)

Fig. 9

Effect of the evaluated product 2,3,5,4' - tetrahydroxystilbene 2-O-β-D-glucoside on melanin production in culture of human melanocytes. Data represent the mean ± standard deviation of 3 repetitions (Anova, Bonferroni)

Fig. 10

Effect of the evaluated products Copper Compound and 2,3,5,4' - tetrahydroxystilbene 2-O-B-D-glucoside on melanin production in culture of human melanocytes. Data represent the mean ± standard deviation of 3 repetitions (Anova, Bonferroni)

**P<0.01 compared with basal control;
*P<0.05 compared with basal control.

Copper Compound (mg/ml) +
2,3,5,4' – TETRAHYDROXYSTILBENE
2-O-B-D-GLUCOSIDE (0,13%)

Fig. 11

The appearance of the solutions after 40 days of storage

Fig. 12

Chromatograms at 280 nm of samples No.1 (25°C) and No.2 (40°C)

Fig. 13

EP 4 751 705 A1

# EP 4 751 705 A1

**EUROPEAN SEARCH REPORT**

Application Number

EP 24 21 6436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/164242 A1 (YU WEN-JEN [TW] ET AL) 28 June 2012 (2012-06-28) * paragraphs [0027], [0049]; claims; figure 11; examples 1,6 * | 1-11,14, 15 | INV. A61K8/19 A61K8/49 A61K8/64 A61Q5/00 A61K8/60 A61K31/7034 A61K33/34 A61P17/00 |
| Y | CN 104 257 564 A (ZHANG YUYUAN) 7 January 2015 (2015-01-07) * claims; examples * | 1-11,14, 15 | |
| Y | CN 107 427 437 A (OREAL) 1 December 2017 (2017-12-01) * paragraph [0001]; claims; examples * | 1-11,14, 15 | |
| Y | CN 114 712 259 A (NOX BELLCOW COSMETICS CO LTD) 8 July 2022 (2022-07-08) * claims; examples * | 1-11,14, 15 | |
| Y | US 2022/151912 A1 (BELOUS ELENA YUR'EVNA [RU]) 19 May 2022 (2022-05-19) * claims; examples * | 1-11,14, 15 | |
| Y | CN 101 606 988 A (GUIZHOU BAIHUA MEDICAL & PHARM [CN]) 23 December 2009 (2009-12-23) * claims; examples * | 12,13 | TECHNICAL FIELDS SEARCHED (IPC) A61K A61P |
| Y | CN 105 055 203 A (YANG BIN) 18 November 2015 (2015-11-18) * "Background technique"; claims * | 12,13 | |
| Y | CA 2 632 542 A1 (PROCTER & GAMBLE [US]; ARCH CHEM INC [US]) 4 January 2001 (2001-01-04) * claims; examples * | 12,13 | |

The present search report has been drawn up for all claims

| Place of search | Date of completion of the search | Examiner |
|---|---|---|
| Munich | 20 May 2025 | Moutafidou, Nikoleta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 6436

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2025

| Patent document cited in search report | | Publication date | Patent family member(s) | | Publication date |
|---|---|---|---|---|---|
| US 2012164242 | A1 | 28-06-2012 | TW | 201225988 A | 01-07-2012 |
| | | | US | 2012164242 A1 | 28-06-2012 |
| CN 104257564 | A | 07-01-2015 | NONE | | |
| CN 107427437 | A | 01-12-2017 | CN | 107427437 A | 01-12-2017 |
| | | | WO | 2016101259 A1 | 30-06-2016 |
| CN 114712259 | A | 08-07-2022 | NONE | | |
| US 2022151912 | A1 | 19-05-2022 | CN | 113557007 A | 26-10-2021 |
| | | | EP | 3954356 A2 | 16-02-2022 |
| | | | US | 2022151912 A1 | 19-05-2022 |
| | | | WO | 2020139145 A2 | 02-07-2020 |
| CN 101606988 | A | 23-12-2009 | NONE | | |
| CN 105055203 | A | 18-11-2015 | NONE | | |
| CA 2632542 | A1 | 04-01-2001 | AT | E424181 T1 | 15-03-2009 |
| | | | AU | 5884600 A | 31-01-2001 |
| | | | BR | 0011852 A | 30-04-2002 |
| | | | CA | 2376803 A1 | 04-01-2001 |
| | | | CA | 2632542 A1 | 04-01-2001 |
| | | | CN | 1414846 A | 30-04-2003 |
| | | | EP | 1189581 A1 | 27-03-2002 |
| | | | EP | 2082723 A1 | 29-07-2009 |
| | | | ES | 2322688 T3 | 25-06-2009 |
| | | | ES | 2599027 T3 | 31-01-2017 |
| | | | HK | 1044489 A1 | 25-10-2002 |
| | | | JP | 5450942 B2 | 26-03-2014 |
| | | | JP | 2003503333 A | 28-01-2003 |
| | | | JP | 2007284459 A | 01-11-2007 |
| | | | KR | 20020057808 A | 12-07-2002 |
| | | | MX | PA02000295 A | 21-05-2004 |
| | | | WO | 0100151 A1 | 04-01-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20120048830 A

- WO 2020089216 A1

**Non-patent literature cited in the description**

- *CHEMICAL ABSTRACTS*, 82373-94-2
- **PANHARDS** ; **LOZANO I** ; **LOUSSOUARNG**. Greying of the human hair: a worldwide survey, revisiting the '50' rule of thumb.. *Br J Dermatol.*, October 2012, vol. 167 (4), 865-73
- **PARKAM** ; **KHANS** ; **RAWNSLEYJ**. Hair Biology: growth and Pigmentation.. *Facial Plast Surg Clin North Am*, November 2018, vol. 26 (4), 415-24
- **KUMARAB** ; **SHAMIMH** ; **NAGARAJUU**. Premature Graying of Hair: review with Updates.. *Int J Trichology.*, September 2018, vol. 10 (5), 198-203
- Hair Pigmentary Unit. **TOBIND.** Aging Hair. Verlag Berlin Heidelberg: Springer, 2010, 77-89
- **TOBIN DJ** ; **PAUSR**. Graying: gerontobiology of the hair follicle pigmentary unit.. *Exp Gerontol.*, January 2001, vol. 36 (1), 29-54
- **JO SJ** ; **PAIKSH** ; **CHOI JW** ; **LEE JH** ; **CHOS** ; **KIMKH et al.** Hair graying pattern depends on gender, onset age and smoking habits.. *Acta Derm Venereol.*, March 2012, vol. 92 (2), 160-1
- **SATOS** ; **JITSUKAWAK** ; **SATOH** ; **YOSHINOM** ; **SETAS** ; **ITOS et al.** Segmented heterochromia in black scalp hair associated with iron-deficiency anemia. Canities segmentata sideropaenica.. *Arch Dermatol.*, April 1989, vol. 125 (4), 531-5
- **NOPPAKUNN** ; **SWASDIKULD**. Reversible hyperpigmentation of skin and nails with white hair due to vitamin B12 deficiency. *Arch Dermatol*, August 1986, vol. 122 (8), 896-9
- Relationship between diet, atopy, family history, and premature hair graying.. **ACERE** ; **KAYA ERDO-GANH** ; **IGREKA** ; **PARLAKH** ; **SARACOGLUZ** ; **BILGINM**. J Cosmet Dermatol.. PubMed, 2018, 1473-2130
- **TOBIN DJ**. The cell biology of human hair follicle pigmentation.. *Pigment Cell Melanoma Res.*, February 2011, vol. 24 (1), 75-88
- **STENNKS** ; **PAUSR**. Controls of hair follicle cycling.. *Physiol Rev*, January 2001, vol. 81 (1), 449-94
- The nature of hair pigment.. **FITZPATRICKTB** ; **BRUNETP** ; **KUKITAA**. The Biology of Hair Growth. Academic Press, 1958, 255-303
- **CLINEDJ**. Changes in hair color. *Dermatol Clin.*, April 1988, vol. 6 (2), 295-303

- **SHAFFRALIFC** ; **MCDONAGHAJ** ; **MESSENGER-AG**. Hair darkening in porphyria cutanea tarda.. *Br J Dermatol.*, February 2002, vol. 146 (2), 325-9
- **PINKUSH**. Postinflammatory hair darkening.. *Arch Dermatol.*, August 1960, vol. 82 (2), 263-4
- **VERBOVJ**. Erosive candidiasis of the scalp, followed by the reappearance of black hair after 40 years.. *Br J Dermatol.*, November 1981, vol. 105 (5), 595-8
- **DALGICB** ; **EGRITAS O**. Gray hair and acrodermatitis enteropathica-like dermatitis: an unexpected presentation of cystic fibrosis.. *Eur J Pediatr.*, October 2011, vol. 170 (10), 1305-8
- **REDONDO P** ; **GUZMÁN M** ; **MARQUINAM** ; **PRETELM** ; **AGUADOL** ; **LLORETP et al.** Repigmentation of gray hair after thyroid hormone treatment. *Actas Dermosifiliogr.*, November 2007, vol. 98 (9), 603-10
- **SEIBERGM**. Age-induced hair greying - the multiple effects of oxidative stress.. *Int J Cosmet Sci.*, December 2013, vol. 35 (6), 532-8
- **WOOD JM** ; **DECKER H** ; **HARTMANNH** ; **CHA-VANB** ; **ROKOS H** ; **SPENCER JD et al.** Senile hair graying: H2O2-mediated oxidative stress affects human hair color by blunting methionine sulfoxide repair.. *FASEB J.*, July 2009, vol. 23 (7), 2065-75
- **AKIN BELLIA** ; **ETGUF** ; **OZBAS GOKS** ; **KARAB** ; **DOGANG**. Risk Factors for Premature Hair Graying in Young Turkish Adults. *Pediatr Dermatol.*, July 2016, vol. 33 (4), 438-42
- **TRÜEBRM**. Association between smoking and hair loss: another opportunity for health education against smoking?. *Dermatology.*, 2003, vol. 206 (3), 189-91
- **NIU C** ; **AISA HA**. Upregulation of melanogenesis and tyrosinase activity: Potential agents for vitiligo.. *Molecules*, 2017, vol. 22, 1303-1305
- **D'ISCHIA M** ; **WAKAMATSU K** ; **NAPOLITANO A** ; **BRIGANTI S** ; **GARCIA-BORRON JC** ; **KOVACS D** ; **MEREDITH P** ; **PEZZELLA A** ; **PICARDO M** ; **SARNA T et al.** Melanins and melanogenesis: Methods, standards, protocols.. *Pigment Cell Melanoma Res.*, 2013, vol. 26, 616-633

- **SLOMINSKI A** ; **ZMIJEWSKI MA** ; **PAWELEK J.** L-tyrosine and L-di-hydroxyphenylalanine as hormone-like regulators of melanocyte functions.. *Pigment Cell Melanoma Res.*, 2012, vol. 25, 14-27
- **PILLAIYAR T** ; **MANICKAM M** ; **NAMASIVAYAM V**. Skin whitening agents: Medicinal chemistry perspective of tyrosinase inhibitors.. *J Enzyme Inhib Med Chem.*, 2017, vol. 32, 403-425
- **HOLLINGER JC** ; **ANGRA K** ; **HALDER RM**. Are natural ingredients effective in the management of hyperpigmentation? A systematic review. *J Clin Aesthet Dermatol.*, 2018, vol. 11, 28-37
- **CARUNTU C** ; **BODA D** ; **CONSTANTIN C** ; **CARUNTU A** ; **NEAGU M**. Catecholamines increase in vitro proliferation of murine B16F10 melanoma cells. *Acta Endocrinol (Copenh)*, 2014, vol. 10, 545-558
- **DIACONEASA A** ; **BODA D** ; **SOLOVAN C** ; **ENESCU DM** ; **VILCEA AM** ; **ZURAC S**. Histopathologic features of Spitzoid lesions in different age groups.. *Rom J Morphol Embryol.*, 2013, vol. 54, 51-62
- **HASEGAWA T**. Tyrosinase-expressing neuronal cell line as in vitro model of Parkinson's disease.. *Int J Mol Sci.*, 2010, vol. 11, 1082-1089
- **ZURAC S** ; **NEAGU M** ; **CONSTANTIN C** ; **CIOPLEA M** ; **NEDELCU R** ; **BASTIAN A** ; **POPP C** ; **NICHITA L** ; **ANDREI R** ; **TEBEICA T et al.** Variations in the expression of TIMP1, TIMP2 and TIMP3 in cutaneous melanoma with regression and their possible function as prognostic predictors.. *Oncol Lett.*, 2016, vol. 11, 3354-3360
- **PAVITHRAN K.** Puvasol Therapy in Premature Greying of Hair.. *Indian J Dermatol Venereol Leprol.*, March 1986, vol. 52 (2), 74-5
- **ROBINSON A** ; **JONES W**. Changes in scalp hair after cancer chemotherapy.. *Eur J Cancer Clin Oncol.*, January 1989, vol. 25 (1), 155-6
- **WARD PD** ; **MILLER HL** ; **SHIPMAN AR**. A case of repigmentation and curling of hair on acitretin therapy. *Clin Exp Dermatol*, January 2014, vol. 39 (1), 91-2
- **SECKIN D** ; **YILDIZ A**. Repigmentation and curling of hair after acitretin therapy.. *Australas J Dermatol.*, August 2009, vol. 50 (3), 214-6
- **VESPER JL** ; **FENSKE NA**. Hair darkening and new growth associated with etretinate therapy.. *J Am Acad Dermatol.*, May 1996, vol. 34 (5), 860
- **KAVAK A** ; **AKCAN Y** ; **KORKMAZ U**. Hair repigmentation in a hepatitis C patient treated with interferon and ribavirin.. *Dermatology.*, 2005, vol. 211 (2), 171-2
- Canities-reversal with chemotherapy.. **BABU KG** ; **RASHESHYAM D** ; **LALITHA N**. J Assoc Physicians India. PubMed, August 1995, vol. 43, 577
- **LOVERING S** ; **MIAO W** ; **BAILIE T** ; **AMATO D**. Hair repigmentation associated with thalidomide use for the treatment of multiple myeloma.. *BMJ Case Rep.*, July 2016
- Repigmentation of hair following adalimumab therapy.. **TINTLE SJ** ; **DABADE TS** ; **KALISH RA** ; **ROSMARIN DM**. Dermatol Online J.. PubMed, June 2015, vol. 21, 1087-2108
- **DASANU CA** ; **MITSIS D** ; **ALEXANDRESCU DT**. Hair repigmentation associated with the use of lenalidomide: graying may not be an irreversible process!. *J Oncol Pharm Pract.*, June 2013, vol. 19 (2), 165-9
- **KHALED A** ; **TROJJET S** ; **ZEGLAOUI F** ; **FAZAA B** ; **KAMOUN MR**. Repigmentation of the white hair after systemic corticosteroids for bullous pemphigoid.. *J Eur Acad Dermatol Venereol.*, August 2008, vol. 22 (8), 1018-20
- **SADIGHHA A** ; **ZAHED GM**. Hair darkening after treatment with cyclosporin in a patient with psoriasis.. *J Eur Acad Dermatol Venereol.*, November 2008, vol. 22 (10), 1239-41
- **REBORA A** ; **DELMONTE S** ; **PARODI A**. Cyclosporin A-induced hair darkening.. *Int J Dermatol.*, March 1999, vol. 38 (3), 229-30
- **ETIENNE G** ; **CONY-MAKHOUL P** ; **MAHON FX**. Imatinib mesylate and gray hair.. *N Engl J Med.*, August 2002, vol. 347 (6), 446
- **CHENG YP** ; **CHEN HJ** ; **CHIU HC** ; **CHEN H.** Erlotinib-induced hair repigmentation.. *Int J Dermatol.*, January 2014, vol. 53 (1), e55-7
- **ALEXANDRESCU DT** ; **KAUFFMAN CL** ; **DASANU CA**. Persistent hair growth during treatment with the EGFR inhibitor erlotinib. *Dermatol Online J.*, March 2009, vol. 15 (3), 4
- **BELLANDI S** ; **AMATO L** ; **CIPOLLINI EM** ; **ANTIGA E** ; **BRANDINI L** ; **FABBRI P**. Repigmentation of hair after latanoprost therapy.. *J Eur Acad Dermatol Venereol.*, December 2011, vol. 25 (12), 1485-7
- **HAMPSON JP** ; **DONNELLY A** ; **LEWIS-JONES MS** ; **PYE JK**. Tamoxifen-induced hair colour change.. *Br J Dermatol.*, March 1995, vol. 132 (3), 483-4
- **REYNOLDS NJ** ; **CROSSLEY J** ; **FERGUSON I** ; **PEACHEY RD**. Darkening of white hair in Parkinson's disease.. *Clin Exp Dermatol.*, July 1989, vol. 14 (4), 317-8
- **DIZDAR O. et al.** Adjuvant use of epidermal growth factor receptor kinase inhibitors may prevent bone metastases in patients with non-small cell lung carcinoma. *Journal of Thoracic Oncology*, 2007, vol. 2 (12), 1136
- **GRAINGER KM**. Pigmentation in Parkinson's disease treated with levodopa.. *Lancet.*, January 1973, vol. 1 (7794), 97-8
- **PASRICHA J.** Successful treatment of gray hairs wtih high dose calcium pantothenate.. *Indian J Dermatol Venereol Leprol.*, 1981, vol. 47 (6), 311-3

- Effect of Grey Hair Evulsion on the response to calcium pantothenate in premature grey hairs.. **PASRICHAJS**. Indian J Dermatol Venereol Leprol.. PubMed, March 1986, vol. 52, 77-80
- **SIEVE B.** *Clinical Achromotrichia. Science*, 1941, vol. 94 (2437), 257-258
- **ZARAFONETISCJ**. Darkening of gray hair during para-aminobenzoic acid therapy.. *J Invest Dermatol.*, December 1950, vol. 15 (6), 399-401
- **BRANDALEONE H** ; **MAINE** ; **STEELEJ**. The effect of calcium pantothenate and para-aminobenzoic acid on gray hair.. *Exp Biol Med.*, 1943, vol. 53 (1), 47-9
- **PHILIPM** ; **SAMSON JF** ; **SIMIPS**. Clofazimine-induced Hair Pigmentation.. *Int J Trichology.*, July 2012, vol. 4 (3), 174-5
- **READ GM**. Verapamil and hair colour change.. *Lancet.*, December 1991, vol. 338 (8781), 1520
- *Drug Information*, 2019, www.uptodate.com.
- **CHRISTOPHT** ; **MÜLLER-RÖVERS** ; **AUDRINGH** ; **TOBIN DJ** ; **HERMESB** ; **COTSARELIS G et al.** The human hair follicle immune system: cellular composition and immune privilege.. *Br J Dermatol.*, May 2000, vol. 142 (5), 862-73
- **BSYM** ; **DIEHLJ** ; **LEVINSPC**. Promising alternative clinical uses of prostaglandin F2α analogs: beyond the eyelashes.. *J Am Dermatology.*, 2015, vol. 72 (4), 712-6
- **CHAVAN D**. Reversal of Premature Hair Graying Treated with a Topical Formulation Containing α-Melanocyte-Stimulating Hormone Agonist (Greyverse Solution 2%). *International Journal of Trichology.*, 2022, vol. 14 (6), 207-209
- **ZEQUN JIANG** ; **JIMIN XU** ; **MINGHAI LONG** ; **ZHIMING TU** ; **GUANGXIAO YANG** ; **GUANGYUAN HE**. 2, 3, 5, 4'-tetrahydroxystilbene-2-O-β-D-glucoside (THSG) induces melanogenesis in B16 cells by MAP kinase activation and tyrosinase upregulation.. *Life Sciences*, 2009, vol. 85, 345-350
- **LIVERTOX**. Clinical and Research Information on Drug-Induced Liver Injury [Internet]. Bethesda (MD). *National Institute of Diabetes and Digestive and Kidney Diseases*, 2012, https://www.ncbi.nlm.nih.gov/books/NBK548795/
- **DONG X** ; **FU J** ; **YIN X** ; **CAO S** ; **LI X** ; **LIN L** ; **HUYILIGEQI**. Ni J. Emodin: A Review of its Pharmacology, Toxicity and Pharmacokinetics.. *Phytother Res.*, 18 May 2016, vol. 30 (8), 1207-18
- **PYO HK** ; **YOO HG** ; **WON CH** ; **LEE SH** ; **KANG YJ** ; **EUN HC** ; **CHO KH** ; **KIM KH**. The effect of tripeptide-copper complex on human hair growth in vitro.. *Arch Pharm Res.*, July 2007, vol. 30 (7), 834-9
- **MILLINGTON KR** ; **MARSH JM**. UV damage to hair and the effect of antioxidants and metal chelators.. *Int J Cosmet Sci.*, 11 March 2020, vol. 42 (2), 174-184
- **KIM S C.J.** ; **CHENG T** ; **GINDULYTE A** ; **HE J** ; **HE S** ; **LI Q** ; **SHOEMAKER BA** ; **THIESSEN PA** ; **YU B** ; **ZASLAVSKY L**. PubChem 2023 update.. *Nucleic Acids Res.*, 2023, vol. 6 (51), 1373
- **NEESE F**. The ORCA program system-Version 5.0. *WIREs Computational Molecular Science*, 2022, vol. 12 (5), e1606
- **ADAMO C.** ; **BARONE V**. Toward reliable density functional methods without adjustable parameters: The PBE0 model. *The Journal of Chemical Physics.*, 1999, vol. 110 (13), 6158
- **GRIMME S.** ; **EHRLICH S.** ; **GOERIGK L.** Effect of the damping function in dispersion corrected density functional theory. *Journal of Computational Chemistry*, 2011, vol. 32 (7), 1456
- **WEIGEND F.** ; **AHLRICHS R.** Balanced basis sets of split valence, triple zeta valence and quadruple zeta valence quality for H to Rn: Design and assessment of accuracy. *Physical Chemistry Chemical Physics*, 2005, vol. 7 (18), 3297